# EUROPEAN PATENT SPECIFICATION

(11) **EP 0 748 399 B1**
(45) Date of publication and mention of the grant of the patent: **15.05.2002**
(21) Application number: 95911651.8
(22) Date of filing: 08.02.1995
(51) Int. Cl.: D01D 5/40, D04H 1/56, D04H 1/42, A61L 15/00, D01F 6/62

(54) **STIRRING PROCESSES FOR PREPARING BIODEGRADABLE FIBRILS**
RÜHRVERFAHREN ZUR HERSTELLUNG BIOLOGISCH ABBAUBARER FIBRILLEN
PROCEDES A AGITATION PERMETTANT DE PRODUIRE DES FIBRILLES BIODEGRADABLES

(30) Priority: 28.02.1994 US 203289
(43) Date of publication of application: 18.12.1996
(73) Proprietor: THE PROCTER & GAMBLE COMPANY, Cincinnati, Ohio 45202 (US)
(72) Inventor: NODA, Isao, Fairfield, OH 45014 (US); LAMPE, Reinhold, August, Okeana, OH 45053 (US); SATKOWSKI, Michael, Matthew, Oxford, OH 45056 (US)
(74) Representative: Hirsch, Uwe Thomas
(86) International application number: US9501652
(87) International publication number: WO9523250

(56) References cited:
- EP-A- 0 177 207
- EP-A- 0 272 902
- GB-A- 2 243 327
- US-A- 2 988 782

## Description

### TECHNICAL FIELD

The present invention relates to the processing of biodegradable polymers and products comprising biodegradable polymers. In particular, the present invention relates to the processing of biodegradable polymers into fibrils, which can be further processed into nonwoven fabrics.

### BACKGROUND OF THE INVENTION

Polymers find uses in a variety of plastic articles including films, sheets, fibers, foams, molded articles, adhesives and many other specialty products. The majority of this plastic material ends up in the solid waste stream, headed for rapidly vanishing and increasingly expensive landfill space. While some efforts at recycling have been made, the nature of polymers and the way they are produced and converted to products limits the number of possible recycling applications. Repeated processing of even pure polymers results in degradation of material and consequently poor mechanical properties. Different grades of chemically similar plastics (e.g., polyethylenes of different molecular weights, as used in milk jugs and grocery sacks) mixed upon collection can cause processing problems that make the reclaimed material inferior or unusable.

Absorbent article applications such as diapers, sanitary napkins, pantiliners and the like, involve several different types of plastics. In these cases, recycling is particularly costly because of the difficulty in separating the different components. Disposable products of this type generally comprise some sort of fluid-pervious topsheet material, an absorbent core, and a fluid-impervious backsheet material. Such absorbent structures are typically prepared using, for example, topsheet materials prepared from woven, nonwoven, or porous formed-film polyethylene or polypropylene materials. Backsheet materials typically comprise flexible polyethylene sheets. Absorbent core materials typically comprise wood pulp fibers or wood pulp fibers in combination with absorbent gelling materials.

A conventional disposable absorbent product is already to a large extent compostable. A typical disposable diaper, for example, consists of about 80% of compostable materials, e.g., wood pulp fibers, and the like. In the composting process soiled disposable absorbent articles are shredded and commingled with organic waste prior to the composting per se. After composting is complete, the non-compostable particles are screened out. In this manner even today's absorbent articles can successfully be processed in commercial composting plants. See for example GB 2 243 327 disclosing biodegradable, liquid imperious sheet laminate.

Nevertheless, there is a need for reducing the amount of non-compostable materials in disposable absorbent articles. There is a particular need to replace polyethylene and polypropylene topsheets in absorbent articles with liquid pervious compostable material.

In addition to being compostable, materials used for topsheets must satisfy many other performance requirements for satisfying the end user's needs. For example, these materials must be capable of being processed to provide substrates that are comfortable to touch. In addition, topsheets must possess sufficient properties such as impact strength, moisture transmission, etc.

Certain biodegradable resins are well known, but often do not have good fiber or film forming properties. See for example US 2 988 782 which describes a process to provide useful particles of a soluble synthetic polymer to produce sheet-like structures. Polymer resins comprising poly(hydroxybutyrate) (PHB) are particularly ill-suited for fiber and film formation. These resins are typically of slow crystallization rates, have low melt viscosity, degrade near their melt temperatures and are rigid when solidified. Accordingly, such resins often cannot be processed by conventional means of film or filament formation. Indeed, when formed into films, biodegradable resins comprising PHB are generally not flexible. The inflexibility of such films hinders their use in absorbent articles, particularly when used as topsheets, as these materials are in direct contact with the skin of the wearer.

For the foregoing reasons, there is a continuing need for biodegradable materials that can be used in absorbent articles. In particular, there is a need for a biodegradable substrate which is flexible and durable, but also provides a comfortable texture when used in, for example, absorbent articles.

It is an object of the present invention to provide biodegradable fibrils that can be processed to give flexible, nonwoven fabrics which are soft and clothlike to the touch. A further object of the invention is to provide a process for making such biodegradable fibrils.

### SUMMARY OF THE INVENTION

The present invention encompasses a process for making biodegradable fibrils from one or more biodegradable resins. The processes are particularly useful in forming fibrils from resins comprising PHB, but can also be utilized with other biodegradable resins. The resins used in the process are biodegradable homopolymers, copolymers or mixtures thereof. The process of the present invention uses a stirred nonsolvent to prepare biodegradable fibrils. In particular, this process involves preparing biodegradable fibrils from one or more biodegradable homopolymeric or copolymeric resins, the process comprising: a) forming a liquid resin mixture by solvating the biodegradable resin or resins; and b) introducing the liquid resin mixture to a nonsolvent while the nonsolvent is stirred. Said process is characterized in that the liquid resin mixture comprises a biologically produced aliphatic polyester. The resulting fibrils can be collected on a suitable collecting device, and are then processed according to their desired end use.

### DETAILED DESCRIPTION

### Definitions

The following is a list of definitions for terms used herein.

"Alkenyl" means a carbon-containing chain which may be monounsaturated (i.e., one double bond in the chain) or polyunsaturated (i.e., two or more double bonds in the chain); straight or branched; and substituted (mono- or poly-) or unsubstituted.

"Alkyl" means a saturated carbon-containing chain which may be straight or branched; and substituted (mono- or poly-) or unsubstituted.

"Biodegradable" means a material capable of being broken down into small chemical subunits which can be utilized in the food chain through naturally acting and/or environmentally safe enzymes, bacteria, spores and the like. Preferably, the material is capable of being broken down into water and CO₂.

A "biodegradable polymer" is any polymer that is biodegradable, as that term is defined herein.

"Compostable" means a material that meets the following three requirements: (1) the material is capable of being processed in a composting facility for solid waste; (2) if so processed, the material will end up in the final compost; and (3) if the compost is used in the soil, the material will ultimately biodegrade in the soil.

The requirement that the material ends up in the final compost typically means that it undergoes a form of degradation in the composting process. Typically, the solid waste stream will be subjected to a shredding step in an early phase of the composting process. As a result, materials will be present as shreds rather than a sheet. In the final phase of the composting process, the finished compost will be subjected to a screening step. Typically, the polymer shreds will not pass through the screens if they have retained the size they had immediately after the shredding step. The compostable materials of the present invention will have lost enough of their integrity during the composting process to allow partially degraded shreds to pass through the screens. However, it is conceivable that a composting facility might subject the solid waste stream to a very rigorous shredding and a rather coarse screening, in which case nondegradable polymers like polyethylene would meet requirement (2). Therefore, meeting requirement (2) is not enough for a material to be compostable within the present definition.

What distinguishes the compostable material as defined herein from material like polyethylene is requirement (3), that the material ultimately biodegrade in the soil. This biodegradability requirement is not essential to the composting process or the use of composting soil. Solid waste and the compost resulting therefrom may contain all kinds of nonbiodegradable materials, for example, sand. However, to avoid a build up of man-made materials in the soil, it is required herein that such materials be fully biodegradable. By the same token, it is not at all necessary that this biodegradation be fast. As long as the material itself and intermediate decomposition products are not toxic or otherwise harmful to the soil or crops, it is fully acceptable that their biodegradation takes several months or even years, since this requirement is present only to avoid an accumulation of man-made materials in the soil.

"Copolymer" and "copolymeric" mean a polymer consisting of two or more different monomeric units.

"Fibrils" means short, fine fibers. The term fibril is discussed in detail herein below.

"Homopolymer" and "homopolymeric" mean a polymer consisting of the same repeating monomeric unit.

"Nonsolvent" means a liquid or liquid mixture in which the biodegradable resins of the present invention are not completely soluble. Use of the term nonsolvent is discussed in detail below.

"Solvating" means forming an aggregate that comprises a solute ion or molecule with one or more solvent molecules, to form a single phase mixture.

### Biodegradable Resins

Biodegradable fibrils of the present invention comprise one or more biodegradable polymers or copolymers. The biodegradable resins useful in the present invention can be any resin which is capable of biodegradation. Resins useful herein may be either biologically or synthetically produced. Furthermore, the resins may be either homopolymeric or copolymeric. As used herein, the terms "resin" and "polymer" include both homopolymeric and copolymeric biodegradable polymers. Furthermore, although stated in the singular, these terms include a plurality of "resins" and "polymers".

Biologically produced biodegradable resins include aliphatic polyesters. Specific examples include poly(3-hydroxybutyrate) (PHB), a polymer made of monomeric units having a structure according to Formula I 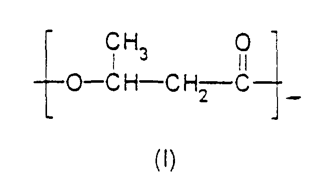 and poly(3-hydroxybutyrate-co-3-hydroxyvalerate) (PHBV), a copolymer which has two randomly repeating monomer units (RRMU) wherein the first RRMU has a structure according to Formula (I) and the second RRMU has a structure according Formula (II) 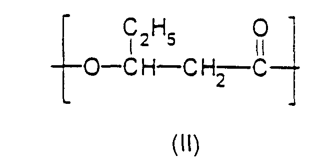 Such polyesters, and methods for their preparation, are described in U.S. Patent 4,393,167, Holmes et al., issued July 12, 1993 and U.S. Patent 4,880,592, Martini et al., issued November 14, 1989, both of which are incorporated by reference herein. Because of their poor film-forming properties, the processes of the present invention are particularly useful for forming nonwoven substrates from these resins. Copolymers of PHBV are commercially available from Imperial Chemical Industries under the tradename BIOPOL. An overview of BIOPOL technology is provided in BUSINESS 2000+ (Winter, 1990).

US Patent 5 618 855 titled BIODEGRADABLE COPOYMERS AND PLASTIC ARTICLES COMPRISING BIODEGRADABLE COPOLYMERS, to Noda, which discloses novel biodegradable copolymers, and methods for making the copolymers, that are also useful in the present invention, is incorporated herein by reference. Briefly, that application describes biodegradable polyhydroxyalkanoate (PHA) copolymers comprising at least two RRMUs wherein the first RRMU has the structure 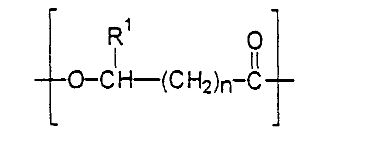 wherein R¹ is H, or C₁ or C₂ alkyl, and n is 1 or 2; the second RRMU has the structure 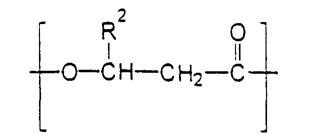 wherein R² is a C₄-C₁₉ alkyl or alkenyl; and wherein at least 50% of the RRMUs have the structure of the first RRMU.

US Patent 5 536 564 to Noda, which discloses novel biodegradable copolymers, and methods for making the copolymers, that are also useful in the present invention, is incorporated herein by reference. Briefly, that application describes biodegradable (PHA) copolymers comprising at least two RRMUs wherein the first RRMU has the structure 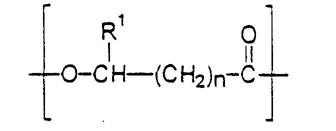 wherein R¹ is H or C₂ alkyl, and n is 1 or 2; the second RRMU has the structure 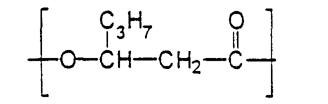 and wherein at least 50% of the RRMUs have the structure of the first RRMU.

US Patent 5 502 116 to Noda, which discloses biodegradable copolymers that are also useful in the present invention, is incorporated herein by reference. Briefly, that application describes biodegradable copolymers, wherein the copolymers comprise at least two RRMUs wherein the first RRMU monomer unit has the structure 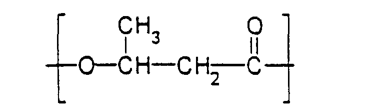 and the second RRMU has the structure 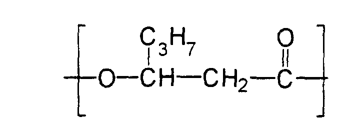 wherein at least 50% of the RRMUs have the structure of the first RRMU.

Other biodegradable resins useful in the present invention include synthetically produced resins. Such synthetically produced resins include aliphatic polyesters, which, along with the methods for making such resins, are known in the art. Examples of such synthetic polyester resins include polylactide (PL), which has a structure according to Formula III; polydioxanone (PDO), which has a structure according to Formula IV; and polycaprolactone (PCL), which has a structure according to Formula V. These resins are described in World Patent Publication No. WO 90/01521, published February 22, 1990 by Sinclair et al.; U.S. Patent No. 5,026,589, issued June 25, 1991 to Schechtman; and ENCYCLOPEDIA OF POLYMER SCIENCE AND ENGINEERING, Second Edition, Vol. 2, pp 220-243 (1983); all of which are incorporated by reference herein.

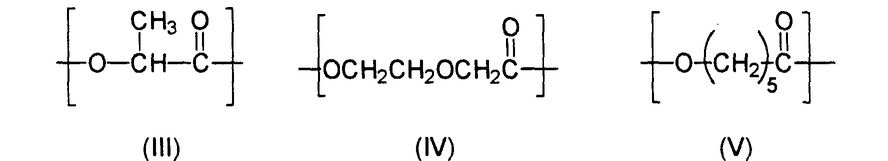

Still other resins useful in the present invention are polyvinyl alcohols and their copolymers. A preferred polyvinyl alcohol copolymer is the copolymer of ethylene and vinyl alcohol, which has two RRMU wherein the first RRMU has the structure 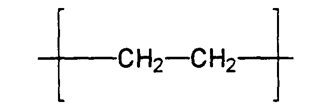 and the second RRMU has the structure

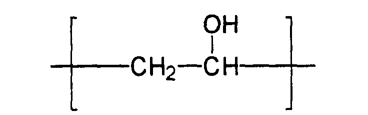

Still other biodegradable resins useful in the present invention are polyethers, such as polyethyleneoxide (PEO) (also referred to polyethyleneglycol), which is also well known in the art.

Another biodegradable resin useful in the present invention is cellulose and its derivatives. An example is cellulose acetate. Cellulose resins, and methods for making them, are well known in the art. See, e.g. ENCYCL. POLYM. SCIENCE AND ENGINEERING, Second Edition, Vol. 3, pp 181-208 (1983).

The biodegradable resins, including those described specifically above, may be used together to make a polymer blend, where said blend is then used to prepare biodegradable fibrils. Furthermore, as indicated, one or more of the resins used to form the polymer blend may be a copolymer. Such blends include, for example : the combination of two polymers selected from the same type of polymer (for example, two biologically produced polyesters) and the combination of polymers selected from two or more different types of polymers (for example, a biologically produced polyester and a polyvinyl alcohol). While not exhaustive, the following are preferred combinations of polymers useful in preparing the biodegradable fibrils of the present invention. PCL and PHB; PCL and PHBV; PHBV and PHB; PHBV and PEO; and PHB and PEO.

### Processes of the Present Invention.

The processes of the present invention are useful for forming biodegradable fibrils. The processes are particularly useful for making fibrils from hard-to-process resins, such as biologically produced polyesters, especially PHB polymer and copolymeric resins comprising hydroxybutyrate comonomer units. Nonetheless, the processes are also useful for making fibrils that do not comprise PHB or copolymeric resins comprising hydroxybutyrate comonomer units.

The process of the present invention uses a stirred nonsolvent to prepare biodegradable fibrils. In particular, this process involves preparing biodegradable fibrils from one or more biodegradable homopolymeric or copolymeric resins, the process comprising : a) forming a liquid resin mixture by solvating the biodegradable resin or resins; and b) introducing the liquid resin mixture to a nonsolvent while the nonsolvent is stirred, characterized in that the liquid resin mixture comprises a biologically produced aliphatic polyester.

With respect to solvating the resin(s) to form the liquid resin mixture, one skilled in the art appreciates that any suitable means for solvating can be used. The appropriate solvent system must be one that allows thorough mixing if a plurality of resins are used, and must in any event not cause decomposition of the resins used. Preferred solvents include, for example, chloroform, methylene chloride, pyridine, trichloroethane, propylene carbonate, and ethylene carbonate. Particularly preferred are chloroform and methylene chloride. The solvents listed are by way of example, and are not intended to limit the scope of the solvents useful in the processes of the present invention.

Any of a variety of additives may be included when forming the liquid resin mixture in step a) of the process of the present invention. Such additives include plasticizers, nucleating agents, binders, fillers, coloring agents and agents that will render the resulting fibrils hydrophobic (for example, where the fibrils will be used to form a topsheet for an absorbent article) or hydrophilic. Suitable nucleating agents include, but are not limited to, boron nitride and talc. Suitable binders include, but are not limited to, natural rubber, synthetic rubber, and PCL. Suitable fillers include, but are not limited to, TiO₂, carbon black and calcium carbonate. Hydrophobic agents include any of those recognized in the absorbent articles field, such as silicone and lower alkyl silicone halides.

Those skilled in the art will recognize that various means for introducing the liquid mixture can be employed. For example, the mixture can be introduced through an orifice by way of gravity, or as the result of a pressurized system. It will also be recognized that conditions under which the introduction of the liquid mixture takes place will affect the characteristics (e.g., fibril length, fibril diameter, fibril stiffness) of the fibrils prepared. Such conditions include, for example, the viscosity of the mixture itself, the solvent system employed in forming the mixture, and the diameter of the orifice through which the liquefied mixture is introduced. The liquid mixture is preferably introduced as a continuous stream through an orifice having a diameter of from about 0.1 mm to about 5 mm. More preferred is where the orifice has a diameter about 0.5 mm to about 2.0 mm. While the rate of delivery of the liquid resin is not critical, a rate of from about 10 g/hour to about 1000 g/hour is preferred. Where desired, a plurality of orifices, each as described herein, may be used to introduce the liquid resin mixture.

The nonsolvent used in step b) will be any solvent or mixture of solvents that will allow the biodegradable resins to solidify from the liquid resin mixture. Accordingly, the resins used in step a) must be at least partially insoluble in the nonsolvent. In addition, the nonsolvent must be at least partly miscible with the solvent(s) used in step a) of the process. Furthermore, the nonsolvent(s) used in step b) may comprise the same material as the solvent system used in step a), but may differ, for example, in pH such that the resins are soluble in the solvent system, but at least partially insoluble in the nonsolvent. The shear forces caused by the stirred nonsolvent will provide fibrils as the resins solidify. Those skilled in the art will recognize that the nonsolvent used will depend on, for example, the solvent system used in step a) of the process, the biodegradable resin(s) and the nature of the fibrils desired. Examples of nonsolvents include methanol, ethanol, acetone, methylethylketone, toluene, and other hydrocarbons such as hexane.

The nonsolvent is maintained at a temperature that will allow solidification of the resin to form fibrils. Preferred is where the nonsolvent is at a temperature of less than about 120°C. More preferred is a temperature of from about 10°C to about 120°C. Most preferred is a temperature of about ambient temperature. Those skilled in the art will recognize that the characteristics (e.g., length, diameter) of the resulting biodegradable fibrils will depend on, for example, the biodegradable resins used, the solvent(s) used in making the liquid resin mixture (when applicable), the nonsolvent used for fibrillation, the shear forces employed, and the rate of delivery of the liquid resin mixture into the nonsolvent.

The stirring of the nonsolvent to which the liquid resin mixture is added allows for the formation of the biodegradable fibrils of the present invention. The desired rate of stirring will depend on, for example, the resins and solvent system used to form the liquid resin mixture, the nonsolvent, and the size and shape of the vessel containing the nonsolvent. Preferred is where the nonsolvent is stirred at a rate such that a laminar flow is created, rather than a turbulent flow. Those skilled in the art will recognize the distinction between laminar and turbulent flow. (See CHEMICAL ENGINEER'S HANDBOOK, Fourth Edition, Chapter 5, p. 16 (1963).)

The formed fibrils can be separated from the solvent and nonsolvent using, for example, filtration and evaporation (including heating).

The fibrils made according to the processes of the present invention are useful in making, for example, biodegradable nonwoven substrates, which can be used in absorbent articles such as diapers and the like.

### Biodegradable Fibrils

The fibrils of the present invention comprise any biodegradable polymeric resin or resins, wherein the fibrils are made according to the processes of the present invention. These resins are discussed in detail above. The fibrils preferably comprise PHB or PHBV.

The fibrils of the present invention are preferably from about 0.5 mm to about 100 mm in length. More preferred are from about 1 mm to about 50 mm in length. Most preferred are fibrils having a length of from about 2 mm to about 10 mm.

The fibrils of the present invention are preferably from about 1µm to about 500 µm in diameter. More preferred are fibrils having a diameter of from about 1µm to about 200 µm. Still more preferred are fibrils from about 5 µm to about 50 µm in diameter.

The preferred ranges listed refer to the fibrils of the invention generally. Preferred fibril dimensions will depend upon the polymeric resins utilized, the specific process employed and the desired end-use of the resulting fibrils.

### Nonwoven Fabrics

The nonwoven fabrics comprise fibrils, made by the processes of the present invention, comprising one or more biodegradable polymers or copolymers. In general, the processes of the present invention will form fibrils that are already in the form of a nonwoven fabric. As such, minimal additional processing may be necessary, depending on the end-use of the nonwoven fabric. Nonetheless, additional steps can be performed to prepare the desired nonwoven.

The fibrils of the present invention are generally shorter than conventional fibers used in fabric-making. Accordingly, if additional processing of the fibrils to give a nonwoven fabric is necessary, preferred is the use of modified paper making techniques. For example, after initial formation using the processes of the present invention, the biodegradable fibrils may be continuously dispersed in a large volume of a nonsolvent (e.g. water) and collected on a moving endless wire screen. Once the nonwoven fabric is collected on the screen, it is transferred to belts or felts and dried on heated drums (see ENCYCLOPEDIA OF POLYMER SCIENCE AND ENGINEERING, Second Edition, Vol. 10, pp 204-226).

Importantly, a nonwoven fabric may comprise biodegradable fibrils made from different resin mixtures. That is, two or more types of fibril, each made using the processes of the present invention, may be combined to form a single nonwoven fabric. While the distinct fibrils will be prepared using the processes of the present invention, the fibrils will subsequently be combined using, for example, the modified paper making techniques described herein.

Furthermore, during the formation of nonwoven fabrics, it is possible to combine with the biodegradable fibrils of the present invention one or more of a variety of natural fibers, such as polysaccharides and polyamides. Thus, the fibrils of the present invention can be combined with cellulose-based fibers, such as wood pulp and cotton, or protein-based fibers such as silk and wool. These naturally occurring fibers can be combined with the fibrils of the present invention using, for example, the paper-making techniques discussed above.

The bonding of fibrils and other components of the nonwoven gives strength to the fabric and influences other properties of the fabric. Both adhesive and mechanical means are used for bonding within the fabric. Mechanical bonding employs the engagement of fibrils by frictional forces. Bonding can also be achieved by chemical reaction, i.e., formation of covalent bonds between binder and fibrils.

### Absorbent Articles

The disposable absorbent articles comprise a water-permeable topsheet, an absorbent core, and a water-impermeable backsheet, wherein the topsheet comprises a nonwoven substrate comprising biodegradable fibrils made by the processes of the present invention.

Biodegradable substrates comprising fibrils of the present invention used as liquid pervious topsheets in absorbent articles of the present invention, such as disposable diapers, typically have a thickness of from about 25 µm to about 1 mm, preferably from about 100 µm to about 500 µm.

In general, the liquid pervious topsheet is combined with a liquid impervious backsheet and an absorbent core positioned between the topsheet and the backsheet. Optionally, elastic members and tape tab fasteners can be included. While the topsheet, the backsheet, the absorbent core and elastic members may be assembled in a variety of well known configurations, a preferred diaper configuration is described generally in U.S. Patent 3,860,003, entitled "Contractible Side Portion for Disposable Diaper" which issued to Kenneth B. Buell on January 14, 1975.

The backsheet can be prepared from either biodegradable or nonbiodegradable materials. Examples of nonbiodegradable backsheets are those made from polypropylene and polyethylene. Examples of biodegradable backsheets are those described in US Patent 5 618 855 titled BIODEGRADABLE COPOYMERS AND PLASTIC ARTICLES COMPRISING BIODEGRADABLE COPOLYMERS; US Patent 5 536 564 and US Patent 5 502 116 all to Noda. To enhance compostability, preferred absorbent articles of the present invention comprise a biodegradable backsheet.

The topsheet is preferably soft-feeling, and non-irritating to the wearer's skin, while at the same time being compostable. Further, the topsheet is liquid pervious, permitting liquids to readily penetrate through its thickness. Preferably, the topsheet is treated with a hydrophobic material to isolate the wearer's skin from liquids in the absorbent core.

Preferably, the topsheet has a real density of from about 18 to about 25 g/m², a minimum dried tensile strength of at least about 400 g/cm in the machine direction, and a wet tensile strength of at least about 55 g/cm in the cross-machine direction.

The topsheet and the backsheet are joined together in any suitable manner. As used herein, the term "joined" encompasses configurations whereby the topsheet is directly joined to the backsheet by affixing the topsheet directly to the backsheet, and configurations whereby the topsheet is indirectly joined to the backsheet by affixing the topsheet to intermediate members which in turn are affixed to the backsheet. In a preferred embodiment, the topsheet and the backsheet are affixed directly to each other in the diaper periphery by attachment means such as an adhesive or any other attachment means known in the art. For example, a uniform, continuous layer of adhesive, a patterned layer of adhesive, or an array of separate lines or spots of adhesive may be used to affix the topsheet to the backsheet.

Tape tab fasteners are typically applied to the back waistband region of the diaper to provide a fastening means for holding the diaper on the wearer. The tape tab fasteners can be any of those well known in the art, such as the fastening tape disclosed in U.S. Patent 3,848,594 issued to Kenneth B. Buell on November 19, 1974. These tape tab fasteners or other diaper fastening means are typically applied near the corners of the diaper.

Preferred diapers have elastic members disposed adjacent the periphery of the diaper, preferably along each longitudinal edge so that the elastic members tend to draw and hold the diaper against the legs of the wearer. The elastic members are secured to the diaper in an contractible condition so that in a normally unrestrained configuration the elastic members effectively contract or gather the diaper. The elastic members can be secured in an contractible condition in at least two ways. For example, the elastic members may be stretched and secured while the diaper is in an uncontracted condition. Alternatively, the diaper may be contracted, for example, by pleating, an elastic member secured and connected to the diaper while the elastic members are in their relaxed or unstretched condition.

The elastic members may take a multitude of configurations. For example, the width of the elastic members may be varied from about 0.25 mm to about 25 mm or more; the elastic members may comprise a single strand of elastic material or the elastic members may be rectangular or curvilinear. Still further, the elastic members may be affixed to the diaper in any of several ways which are known in the art. For example the elastic members may be ultrasonically bonded, heat and pressure sealed into the diaper using a variety of bonding patterns, or the elastic members may simply be glued to the diaper.

The absorbent core of the diaper is positioned between the topsheet and backsheet. The absorbent core may be manufactured in a wide variety of sizes and shapes (e.g., rectangular, hour-glass, asymmetrical, etc.) and from a wide variety of materials. The total absorbent capacity of the absorbent core should, however, be compatible with the designed liquid loading for the intended use of the absorbent article or diaper. Further, the size and absorbent capacity of the absorbent core may vary to accommodate wearers ranging from infants through adults.

A diaper has an hour-glass shaped absorbent core. The absorbent core is preferably an absorbent member comprising a web or batt of airfelt, wood pulp fibers, and/or a particulate absorbent polymeric composition disposed therein.

Other examples of absorbent articles are sanitary napkins designed to receive and contain vaginal discharges such as menses. Disposable sanitary napkins are designed to be held adjacent to the human body through the agency of a garment, such as an undergarment or a panty or by a specially designed belt. Examples of the kinds of sanitary napkins to which the present invention is readily adapted are shown in U.S. Patent 4,687,478, entitled "Shaped Sanitary Napkin With Flaps" which issued to Kees J. Van Tilburg on August 18, 1987, and in U.S. Patent 4,589,876, entitled "Sanitary Napkin" which issued to Kees J. Van Tilburg on May 20, 1986. It will be apparent that the fibrils of the present invention described herein may be used as the liquid pervious topsheet of such sanitary napkins.

In general, sanitary napkins comprise a liquid impervious backsheet, a liquid pervious topsheet, and an absorbent core placed between the backsheet and the topsheet. The topsheet comprises fibrils made by the processes of the present invention. The backsheet may comprise any of the backsheet materials discussed with respect to diapers. The absorbent core may comprise any of the absorbent core materials discussed with respect to diapers.

Importantly, the absorbent articles according to the present invention are biodegradable and/or compostable to a greater extent than conventional absorbent articles which employ materials such as a polyolefin (e.g., a polyethylene) topsheet.

### Textiles

The textiles may be prepared directly from the nonwoven fabrics. Thus, the textiles will comprise as a primary material only the biodegradable nonwoven fabrics.

Alternatively, the textiles can be prepared by combining biodegradable fibrils of the present invention with other fibers, preferably biodegradable fibers such as cotton, rayon, hemp, wool, and silk, to form fabrics, threads or yarns. The textiles can be prepared by using known methods for making textile articles. Such methods are described in KIRK-OTHMER ENCYCLOPEDIA OF CHEMICAL TECHNOLOGY, 3rd edition, Vol. 22, pp 762-768 (1986), which is incorporated by reference herein.

Regardless of the method of preparation, the textiles are far ranging in their application areas. Such applications include, for example, apparel, bedding sheets, upholstery, carpeting, wall covering, tire reinforcement, tenting, filter media, conveyor belts and insulation. The textiles are biodegradable and/or compostable to a greater extent than conventional textiles known in the art.

### EXAMPLE 1

### Process for Making a Nonwoven Fabric Comprising Poly(3-hydroxybutyrate-co-3-hydroxyvalerate)

A 10%-solution of poly(3-hydroxybutyrate-co-3-hydroxyvalerate) with the comonomer content of 8-mol% hydroxyvalerate (BIOPOL, ICI, Billingham UK) in chloroform (J. T. Baker, Phillipsberg NJ) is produced by dissolving 50 g of poly(3-hydroxybutyrate-co-3-hydroxyvalerate) in 450 g of solvent. The solution mixture is poured down through a 5-mL glass pipette with a 3-mm diameter tip into a precipitation bath containing methanol (J. T. Baker, Phillipsberg NJ) which is stirred gently. The polymer solution mixed with the circular flow of methanol instantaneously starts the precipitation and produces a steady stream of a suspension of fine highly elongated fibrils having a diameter between 10 to 500 µm and length from about 3 to about 20 mm. The fibrils are collected to form a continuous nonwoven fabric by filtering off the mixture of chloroform and methanol using a mesh screen. The resulting nonwoven fabric is dried and heat cured in an oven at 120°C for 1 hour.

### EXAMPLE 2

### Process for Making a Nonwoven Fabric Comprising Poly(3-hydroxybutyrate) and Poly(ε-caprolactone)

A 10%-solution of a 9:1 resin mixture of poly(3-hydroxybutyrate) (BIOPOL, ICI, Billingham UK) and poly(ε-caprolactone) (TONE P787, Union Carbide, Danbury CT) in chloroform (J. T. Baker, Phillipsberg NJ) is produced by dissolving a mixture of 45 g of poly(3-hydroxybutyrate) and 5 g of poly(ε-caprolactone) in 450 g of solvent. The solution mixture is poured down through a 5-mL glass pipette with a 3-mm diameter tip into a precipitation bath containing methanol (J. T. Baker, ) which is stirred gently. The polymer solution mixed with the circular flow of methanol instantaneously starts the precipitation and produces a steady stream of a suspension of fine highly elongated fibrils having a diameter between 10 to 500 µm and a length from about 3 mm to 20 mm. The fibrils are collected to form a continuous nonwoven fabric by filtering off the mixture of chloroform and methanol using a mesh screen. The resulting nonwoven fabric is dried and heat cured in an oven at 120°C for 1 hour.

### EXAMPLE 3

### Process for Making a Nonwoven Fabric Comprising Poly(3-hydroxybutyrate-co-3-hydroxyvalerate) and Poly(ε-caprolactone)

A 10%-solution of a 1:1 resin mixture of poly(3-hydroxybutyrate-co-3-hydroxyvalerate) with the comonomer content of 8-mol% hydroxyvalerate (BIOPOL, ICI, Billingham UK) and poly(ε-caprolactone) (TONE P787, Union Carbide, Danbury CT) in chloroform (J. T. Baker, Phillipsberg NJ) is produced by dissolving a mixture of 25 g of poly(3-hydroxybutyrate-co-3-hydroxyvalerate) and 25 g of poly(ε-caprolactone) in 450 g of solvent. The solution mixture is poured down through a 5-mL glass pipette with a 3-mm diameter tip into a precipitation bath containing methanol (J. T. Baker, Phillipsberg NJ) which is stirred gently. The polymer solution mixed with the circular flow of methanol instantaneously starts the precipitation and produces a steady stream of a suspension of fine highly elongated fibrils having a diameter from about 10 to 500 µm and length from about 3 to 20 mm. The fibrils are collected to form a continuous nonwoven fabric by filtering off the mixture of chloroform and methanol using a mesh screen. The resulting nonwoven fabric is dried and heat cured in an oven at 60°C for 1 hour.

### EXAMPLE 4

### Process for Making a Nonwoven Fabric Comprising Poly(3-hydroxybutyrate-co-3-hydroxyvalerate) and Wood Pulp

A 10%-solution of poly(3-hydroxybutyrate-co-3-hydroxyvalerate) with the comonomer content of 12-mol% hydroxyvalerate (BIOPOL, ICI, Billingham UK) in chloroform (J. T. Baker, Phillipsberg NJ) is produced by dissolving 50 g of poly(3-hydroxybutyrate-co-3-hydroxyvalerate) in 450 g of solvent. The solution mixture is poured down through a 5-mL glass pipette with a 3-mm diameter tip into a precipitation bath containing methanol (J. T. Baker, Phillipsberg NJ) which is stirred gently. The polymer solution mixed with the circular flow of methanol instantaneously starts the precipitation and produces a steady stream of a suspension of fine fibrils having a diameter of about 10 to 500 µm and a length of from about 2 to 20 mm. The fibrils are collected by filtering off the mixture of chloroform and methanol using a coarse mesh screen. The fibrils are then resuspended in water with equal weight of wood pulp with the consistency of 1% fibril-pulp mixture. The fibril-pulp mixture is collected to form a continuous nonwoven fabric by filtering off the water using a mesh screen. The resulting nonwoven fabric is dried and heat cured in an oven at 110°C for 30 min.

### EXAMPLE 5

### Compostable Disposable Diaper

A disposable baby diaper is prepared as follows. The dimensions listed are for a diaper intended for use with a child in the 6-10 kilogram size range. These dimensions can be modified proportionately for different size children, or for adult incontinence briefs, according to standard practice.
1. Backsheet: 0.020 - 0.038 mm film consisting of polyethylene (as described in U.S. Patent No. 3,860,003, issued January 14, 1974 to Buell, which is incorporated herein by reference); width at top and bottom 33 cm; notched inwardly on both sides to a width-at-center of 28.5 cm; length 50.2 cm.
2. Topsheet: comprises the nonwoven fabric prepared in Example 1; width at top and bottom 33 cm; notched inwardly on both sides to a width-at-center of 28.5 cm; length 50.2 cm.
3. Absorbent core: comprises 28.6 g of cellulose wood pulp and 4.9 g of absorbent gelling material particles (commercial polyacrylate from Nippon Shokubai); 8.4 mm thick, calendered; width at top and bottom 28.6 cm; notched inwardly at both sides to a width-at-center of 10.2 cm; length 44.5 cm.
4. Elastic leg bands: four individual rubber strips (2 per side); width 4.77 mm; length 370 mm; thickness 0.178 mm (all the foregoing dimensions being in the relaxed state).

The diaper is prepared in standard fashion by positioning the core material covered with the topsheet on the backsheet and gluing.

The elastic bands (designated "inner" and "outer", corresponding to the bands closest to, and farthest from, the core, respectively) are stretched to ca. 50.2 cm and positioned between the topsheet/backsheet along each longitudinal side (2 bands per side) of the core. The inner bands along each side are positioned ca. 55 mm from the narrowest width of the core (measured from the inner edge of the elastic bank). This provides a spacing element along each side of the diaper comprising the flexible topsheet/backsheet material between the inner elastic and the curved edge of the core. The inner bands are glued down along their length in the stretched state. The outer bands are positioned ca. 13 mm from the inner bands, and are glued down along their length in the stretched state. The topsheet/backsheet assembly is flexible, and the glued-down bands contract to elasticize the sides of the diaper.

### EXAMPLE 6

### Compostable Lightweight Pantiliner

A lightweight pantiliner suitable for use between menstrual periods comprises a pad (surface area 117 cm²; SSK air felt 3.0 g) containing 1.0 g of absorbent gelling material particles (commercial polyacrylate; Nippon Shokubai); said pad being interposed between a topsheet according to Example 1 and a backsheet which comprises a 0.03 mm thickness polyethylene film.

### EXAMPLE 7

### Compostable Sanitary Napkin

A catamenial product in the form of a sanitary napkin having two flaps extending outward from its absorbent core is prepared using a pad in the manner of Example 6 (surface area 117 cm²; 8.5 g SSK air felt), per the design of U.S. Patent 4,687,478, Van Tilburg, August 18, 1987. The backsheet and topsheet materials are the same as described in Example 6.

### EXAMPLE 8

### Compostable Nonwoven Fabric

The biodegradable fibrils of Example 1 are collected on a cardboard mat. The mat is moved in a fashion so that a 10 cm x 10 cm area is covered uniformly with fibrils. Collection of fibrils on the mat continues, until there is approximately 0.5 cm thick fibril mat. A wide distribution of fibril lengths are obtained, up to 100 mm in length. Most fibril lengths (over 50%) are in the range of 5 to 20 mm. The mat is then transferred to a Carver Press (Fred S. Carver Inc., Menomonee Falls, WI) and pressed at a 1000 Ib force for 10 minutes at temperature 5°C below the melting temperature of the PHB-V. The resulting nonwoven sheet is removed from the press.

### EXAMPLE 9

### Compostable Disposable Diaper

A disposable baby diaper is prepared as follows. The dimensions listed are for a diaper intended for use with a child in the 6-10 kilogram size range. These dimensions can be modified proportionately for different size children, or for adult incontinence briefs, according to standard practice.
1. Backsheet: 0.020 - 0.038 mm film consisting of a 92:8 poly(3-hydroxybutyrate-co-3-hydroxyoctanoate) copolymer (prepared as described in co-pending application U.S.S.N _, titled BIODEGRADABLE COPOYMERS AND PLASTIC ARTICLES COMPRISING BIODEGRADABLE COPOLYMERS, by Noda, filed January 28, 1994); width at top and bottom 33 cm; notched inwardly on both sides to a width-at-center of 28.5 cm; length 50.2 cm.
2. Topsheet: comprises the nonwoven fabric prepared in Example 1; width at top and bottom 33 cm; notched inwardly on both sides to a width-at-center of 28.5 cm; length 50.2 cm..
3. Absorbent core: comprises 28.6 g of cellulose wood pulp and 4.9 g of absorbent gelling material particles (commercial polyacrylate from Nippon Shokubai); 8.4 mm thick, calendered; width at top and bottom 28.6 cm; notched inwardly at both sides to a width-at-center of 10.2 cm; length 44.5 cm.
4. Elastic leg bands: four individual rubber strips (2 per side); width 4.77 mm; length 370 mm; thickness 0.178 mm (all the foregoing dimensions being in the relaxed state).

The diaper is prepared in standard fashion by positioning the core material covered with the topsheet on the backsheet and gluing.

The elastic bands (designated "inner" and "outer", corresponding to the bands closest to, and farthest from, the core, respectively) are stretched to ca. 50.2 cm and positioned between the topsheet/backsheet along each longitudinal side (2 bands per side) of the core. The inner bands along each side are positioned ca. 55 mm from the narrowest width of the core (measured from the inner edge of the elastic bank). This provides a spacing element along each side of the diaper comprising the flexible topsheet/backsheet material between the inner elastic and the curved edge of the core. The inner bands are glued down along their length in the stretched state. The outer bands are positioned ca. 13 mm from the inner bands, and are glued down along their length in the stretched state. The topsheet/backsheet assembly is flexible, and the glued-down bands contract to elasticize the sides of the diaper.

### EXAMPLE 10

### Compostable Lightweight Pantiliner

A lightweight pantiliner suitable for use between menstrual periods comprises a pad (surface area 117 cm²; SSK air felt 3.0 g) containing 1.0 g of absorbent gelling material particles (commercial polyacrylate; Nippon Shokubai); said pad being interposed between a topsheet according to Example 1 and a backsheet which comprises a 0.03 mm thickness 92:8 poly(3-hydroxybutyrate-co-3-hydroxyoctanoate) copolymer film (prepared as described in co-pending application U.S.S.N _, titled BIODEGRADABLE COPOYMERS AND PLASTIC ARTICLES COMPRISING BIODEGRADABLE COPOLYMERS, by Noda, filed January 28, 1994).

### EXAMPLE 11

### Compostable Sanitary Napkin

A catamenial product in the form of a sanitary napkin having two flaps extending outward from its absorbent core is prepared using a pad in the manner of Example 10 (surface area 117 cm²; 8.5 g SSK air felt), per the design of U.S. Patent 4,687,478, Van Tilburg, August 18, 1987. The backsheet and topsheet materials are the same as described in Example 10.

### EXAMPLE 12

### Compostable Surgical Scrup

Apparel suitable for wearing by surgical staff which can be subsequently disposed of for biodegradation; said apparel comprising the nonwoven fabric of Example 1 sewn in the design of a pullover shirt and the nonwoven fabric of Example 1 sewn in the design of a pair of pants comprising a waist drawstring.

It is understood that the examples and embodiments described herein are for illustrative purposes only and that various modifications or changes in light thereof will be suggested to one skilled in the art and are to be included in spirit and purview of this application and scope of the appended claims.

## Claims

1. A process for preparing biodegradable fibrils from one or more biodegradable homopolymeric or copolymeric resins, said process comprising :
a) forming a liquid resin mixture by solvating the biodegradable resin or resins ; and
b) introducing the liquid resin mixture to a nonsolvent while the nonsolvent is stirred,
**characterized in that** the liquid resin mixture comprises a biologically produced aliphatic polyester.

2. The proces of claim 1, wherein the fibrils are from about 1 mm to about 100 mm in length.

3. The process of claim 1, wherein the fibrils are from about 2 mm to about 10 mm in length and from about 5 µm to about 500 µm in diameter.

4. The process of claim 1, wherein the biologically produced aliphatic polyester is selected from the group consisting of poly(3-hydroxybutyrate), poly(3-hydroxybutyrate-co-3-hydroxyvalerate), or a mixture thereof.

## Patentansprüche

1. Verfahren zur Herstellung bioabbaubarer Fibrillen aus einem oder mehreren bioabbaubaren, homopolymeren oder copolymeren Harzen, wobei das Verfahren umfaßt:
a) Bilden einer flüssigen Harzmischung durch Solvatisieren des bioabbaubaren Harzes oder der Harze; und
b) Einbringen der flüssigen Harzmischung in ein Nichtlösungsmittel, während das Nichtlösungsmittel gerührt wird,
**dadurch gekennzeichnet, daß** die flüssige Harzmischung einen biologisch erzeugten, aliphatischen Polyester umfaßt.

2. Verfahren nach Anspruch 1, wobei die Fibrillen eine Länge von etwa 1 mm bis etwa 100 mm aufweisen.

3. Verfahren nach Anspruch 1. wobei die Fibrillen eine Länge von etwa 2 mm bis etwa 10 mm und einen Durchmesser von etwa 5 µm bis etwa 500 µm aufweisen.

4. Verfahren nach Anspruch 1, wobei der biologisch erzeugte, aliphatische Polyester aus der Poly(3-hydroxybutyrat), Poly(3-hydroxybutyrat-co-3-hydroxyvalerat) oder einer Mischung hiervon umfassenden Gruppe gewählt ist.

## Revendications

1. Procédé de préparation de fibrilles biodégradables à partir d'une ou plusieurs résines homopolymères ou copolymères biodégradables, ledit procédé consistant à :
a) former un mélange résineux liquide en solvatant la ou les résine(s) biodégradable(s) ; et
b) introduire le mélange résineux liquide dans un non-solvant tout en agitant ce non-solvant,
**caractérisé en ce que** le mélange résineux liquide comprend un polyester aliphatique d'origine biologique.

2. Procédé de la revendication 1, dans lequel les fibrilles ont une longueur d'environ 1 mm à environ 100 mm.

3. Procédé de la revendication 1, dans lequel les fibrilles ont une longueur d'environ 2 mm à environ 10 mm, et un diamètre d'environ 5 µm à environ 500 µm.

4. Procédé de la revendication 1, dans lequel le polyester aliphatique d'origine biologique est choisi dans le groupe constitué par le poly(3-hydroxybutyrate), le poly(3-hydroxybutyrate-co-3-hydroxyvalérate), ou un de leurs mélanges.
